**Europäisches Patentamt**

**European Patent Office**

**Office européen des brevets**

(19)

(11) Veröffentlichungsnummer: **0 085 273**
**B1**

(12) **EUROPÄISCHE PATENTSCHRIFT**

(45) Veröffentlichungstag der Patentschrift:
**10.06.87**

(21) Anmeldenummer: **82710004.1**

(22) Anmeldetag: **03.02.82**

(51) Int. Cl.⁴: **A 61 B 17/32**, A 61 B 17/04,
A 61 C 3/02

(54) **Nahtentfernungsschere.**

(43) Veröffentlichungstag der Anmeldung:
**10.08.83 Patentblatt 83/32**

(45) Bekanntmachung des Hinweises auf die Patenterteilung:
**10.06.87 Patentblatt 87/24**

(84) Benannte Vertragsstaaten:
**AT BE CH DE FR GB IT LI LU NL SE**

(56) Entgegenhaltungen:
**DE-C-424 807**
**US-A-1 913 770**
**US-A-3 721 245**
**US-A-3 866 610**
**US-A-4 035 917**
**US-A-4 285 344**

(73) Patentinhaber: **Schäfer, Jürgen, Lange Strasse 3,
D-2860 Osterholz- Scharmbeck (DE)**

(72) Erfinder: **Schäfer, Jürgen, Lange Strasse 3, D-2860
Osterholz- Scharmbeck (DE)**

(74) Vertreter: **Munderich, Paul, Dipl.- Ing., Frankfurter
Strasse 84, D-6466 Gründau- Rothenbergen (DE)**

EP 0 085 273 B1

## Beschreibung

Die Erfindung betrifft eine Nahtentfernungsschere, bestehend aus zwei Scherarmen mit je einem kurzen Funktionsende, d.h. mit einem kurzen Bereich der gelenkbildenden Zusammenführung und einem kurzen Bereich der Schneidenausbildung und einem langen Manipulationsende mit Ösengriff, wobei zwischen den im wesentlichen geradlinig verlaufenden Funktions- und Manipulationsenden U-förmige Umlenkbereiche vorgesehen sind, die winklig bis bogenförmig ausgeführt sind, so daß das Funktionsende zum Manipulationsende um 180° umgelenkt ausgebildet ist, und wobei das Breitenmaß der beiden Scherarme im Bereich des Funktionsendes jeweils um die Hälfte reduziert ausgebildet ist, insbesondere für den Gebrauch in der Mundhöhle.

Eine solche Schere ist durch die Aussage der im Prüfungsverfahren genannten Schrift US-A-4 285 344 bekannt.

Sie betrifft eine chirurgische Schere mit einem kurzen Bereich der gelenkbildenden Zusammenführung einschließlich eines kurzen Funktionsendes, d.h. einschließlich einem kurzen Bereich der Schneidenausbildung und einem langen Manipulationsende, d.h. einem langen Griffbereich.

Die Manipulationsenden und die Schneidenden sind dabei, lageparallel versetzt, in zwei Bewegungsebenen liegend angeordnet. Ein Senkrechtschnitt durch einen im wesentlichen waagrecht liegenden Nahtfaden in Scherenebene ist nicht möglich.

Bei dem Versuch, einen von oben und unten zu führenden Schnitt durch den Nahtfaden zu vollziehen, müßte die Schere entgegen der gezeichneten Darstellung und im Abstand des Maßes der parallelen Versetzung der beiden Bereiche, deren Betätigung in Richtung des Mittelbereiches der Mundhöhle verrückt werden. Dies ist für die Entfernung distaler Nähte praktisch nicht möglich.

Nimmt man z.B. an - in diesem Zusammenhang darf zur Erläuterung der Situation etwas weiter ausgeholt werden -, daß der "Sechs-Jahr-Molar" entfernt wurde, so kann es der Fall sein, daß die zwischen dem "5er" und "7er" entstehende Lücke vernäht werden muß.

Dies gilt insbesondere dann, wenn die buccale Kieferwand hierbei entfernt werden muß.

Hierbei werden die Randlappen in Richtung des Kieferverlaufes von beiden Seiten zwischen "5" und "7" in die entstandene Lücke nach innen gezogen und etwa mittig zwischen diesen beiden Molaren eine Fadenschlinge gesetzt, deren Fadenverlauf im wesentlichen senkrecht zum Nahtverlauf, der in diesem Fall in etwa identisch mit dem Kieferverlauf ist, gerichtet ist.

Die Entfernung dieser breit gesetzten Nahtschlinge wird durch den Hauszahnarzt mit einer geraden Nahtentfernungsschere vorgenommen. Ein korrekter Nahtverschluß wäre jedoch durch den Ansatz von je einer Fadenschlinge vor "7" und einer Fadenschlinge hinter "5" gegeben.

Eine, wenn man bei diesem Beispiel bleibt, unmittelbar hinter dem "5er" gelegte Nahtschlinge ist mit einer geraden Schere praktisch nicht oder aber nur sehr schwer entfernbar.

Das Setzen von nur einer Nahtschlinge zwischen den beiden genannten Molaren ist aber umgekehrt mit einer verlängerten Wundheilung verbunden, da sich beidseits der Nahtschlinge Granulationsgewebe in Form von zwei Granulationsdreiecken bildet, deren Fläche nur durch das Setzen von zwei Nahtschlingen verringert werden könnte.

Eine Einfachnaht erschwert also vielfach die postoperative Wundversorgung durch den Hauszahnarzt.

Beim Entfernen einer, z.B. in einer chirurgischen Klinik, durch mehrere eng gelegte Nahtschlingen gebildeten Naht und der späteren Nachbehandlung in einer Hauszahnarztpraxis ist eine Erschwerung der Situation gegeben.

Der Hauszahnarzt muß dann mit dem ihm zur Verfügung stehenden Instrumentarium auch eine so gebildete Naht entfernen.

Dies ist nicht einfach und erfordert eine hohe Geschicklichkeit, insbesondere bei Entfernung der distal gesetzten Nahtschlinge.

Dies berücksichtigend ist es Aufgabe dieser Erfindung, eine Nahtentfernungsschere nach der eingangs beschriebenen Art zu nennen, mit deren Hilfe schwer zugängliche, insbesondere distal verlegte, im wesentlichen horizontal liegende Nähte, durch einen senkrecht hierzu geführten Schnitt leicht entfernbar sind.

Die erfindungsgemäße Lösung dieser Aufgabe sieht vor,

daß die Umlenkung um eine Achse erfolgt, die senkrecht zur Bewegungsebene beider Ösengriffe verläuft, und

daß der Abstand des beide Scherarme verbindenden Gelenkes zu dem Außenbereich des im Umlenkbereich innenliegenden Scherarmes so gewählt ist,

daß im Umlenkbereich der innenliegende Scherarm im außenliegenden Scherarm, entsprechend der Öffnungsweite des verbindenden Gelenkes, voll verschwenkbar ist.

Durch die U-förmige Umlenkung des durch die beiden ineinanderliegend angeordneten Scherarme gebildeten Funktionsendes, insbesondere in Verbindung mit dessen möglicher Rechts- bzw. Linksabbiegung, wird erreicht, daß Nahtschlingen in jeder praktisch denkbaren Lage, insbesondere auch distal liegende, auf einfache, bequeme Art aus dem Bereich der Wunde entfernt werden können, da die Nahtentfernungsschere mit ihrer umgekehrt gerichteten Spitze, d.h. ihrem Funktionsende,

a) unmittelbar hinter einen Zahn führbar ist und

b) durch die Abbiegung des Funktionsendes entweder nach links oder nach rechts bei verringerter Spreizbeanspruchung für den Patienten in Richtung des Kieferverlaufes geführt werden kann.

Das zulässige Maß zwischen Außen- und Innenscherarm, das durch den Abstand zwischen der an die Umlenkung des inneren Scherarmes geführten Tangente zum Gelenk bestimmt ist, sichert, bei Einhaltung oder geringfügiger Unterschreitung des inneren Maßes der parallel umgelenkten Bereiche des äußeren Scherarmes, die volle Verschwenkbarkeit des inneren Scherarmes.

Eine mögliche Ausbildung sieht vor, daß die Umlenkung des außenliegenden Scherarmes vollständig ist, während die Umlenkung des innenliegenden Scherarmes ein geringfügig reduziertes Winkelmaß gegenüber dem Winkelmaß der Umlenkung des außenliegenden Scherarmes aufweist, und

daß bei zusammengeführten Manipulationsenden und damit bei geschlossenen Funktionsenden, der innere, vom Manipulationsende bis zum Umlenkbereich führende Teil des innenliegenden Scherarmes zum außenliegenden Scherarm einen spitzen, sich in Richtung auf den Umlenkbereich öffnenden Winkel bildet.

Eine einseitige, im Bereich der elastischen Verformung des Manipulationsendes dieser Scherarme liegende Beanspruchung kann sich also nicht auf die Führung der Schneiden zueinander auswirken, da sich diese in jedem Fall gegeneinander abstützen.

Der vorgeschlagene, nur um wenige Winkelgrade sich öffnende spitze Winkel zwischen den Innenflächen der Scherarme ermöglicht eine ungehinderte Verschwenkung der Arme zueinander.

Bei kreisbogenförmigen Umlenkungen ist in aller Regel vorzusehen, daß der Drehpunkt des Gelenkes unmittelbar im Auslaufbereich der zum Funktionsende gerichteten Umlenkbereiche der Scherarme angeordnet ist.

Um die Auswirkungen von im Grunde genommenen mißbräuchlichen Beanspruchungen der Manipulationsenden, d.h. eines Verschiebens der beiden Enden zueinander, zu vermeiden, wird, bei Berücksichtigung des elastischen Verhaltens des Scherarmmaterials vorgeschlagen, daß das Längenmaß der vom Gelenk zum Manipulationsende gerichteten Bereiche der Scherarme ein Vielfaches der Länge der das Funktionsende bildenden Scherarmbereiche aufweist.

Bei Verwendung von üblichem Instrumentenstahl ist vorzusehen, daß das Längenverhältnis der Funktionsenden zu den Manipulationsenden der Scherarme 1:4 bis 1:8 beträgt.

Des weiteren ist vorgesehen, daß, bezogen auf die Bewegungsebene der Ösengriffe, das Funktionsende seitlich abgebogen ist.

In diesem Zusammenhang kann es, im Interesse einer Materialeinsparung, zweckmäßig sein, daß die Funktionsenden unmittelbar nach dem Gelenk auswechsel- bzw. austauschbar ausgebildet sind.

Die vorgeschlagene Lösung wird in vollem Umfang der Aufgabenstellung gerecht.

Die Erfindung wird durch die beigefügte zeichnerische Darstellung einer beispielsweisen Ausbildung einer Nahtentfernungsschere näher erläutert.

Figur 1 zeigt die Schere im Aufriß in geschlossener Darstellung.

Figur 2 zeigt die Schere im Aufriß in geöffneter Darstellung.

Figur 3 zeigt die Schere mit rechts abgebogenem Funktionsende in einer Lage zur Entfernung einer distal gesetzten Nahtschlinge hinter einem 5-ER-Molar.

Die Nahtentferungsschere 1 besteht aus je einem Innenscherarm 2 und einem Außenscherarm 3. Das Manipulationsende 1.1 der Schere 1 wird aus je einem jedem Scherarm 2 und 3 zugeordneten Ösengriff 2.1 und 3.1 gebildet, während das Funktionsende 1.2 aus der Unterschneide 2.2 und der Oberschneide 3.2 besteht.

Die Unterschneide 2.2 weist eine Aufnahmeanformung 2.3 für den vor dem Schnitt von hier aufzunehmenden Faden auf.

Die beiden Scherarme 2 und 3 sind in ihrem Breitenmaß 1.3 im Bereich der gelenkbildenden Zusammenführung 4.1 überführend in den Bereich des Funktionsendes 1.2 in ihrer Breite, und zwar um die Hälfte, reduziert, und durch das Gelenk 4 verbunden.

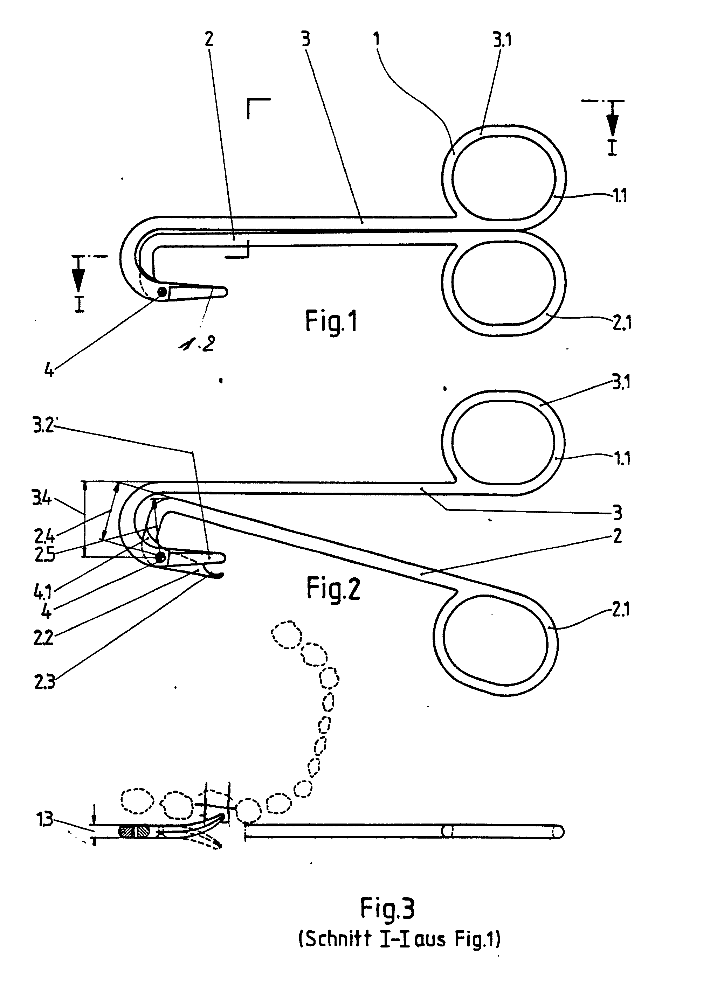

Wie aus Figur 1 zu entnehmen ist, wird bei geschlossener Nahtentfernungsschere 1 ein spitzer Winkel zwischen den zum Manipulationsende führenden Scherarm 2 und 3 gebildet.

Hierbei ist der Außenscherarm 3 vollständig, d.h. um 180° umgelenkt, abgebogen, während der Innenscherarm 2 um das Winkelmaß des zwischen den Scherarmen verbleibenden spitzen Winkels reduziert ist.

Der durch den Abstand des Gelenkes 4 zu einem an dem Außenbereich des Innenscherarmes 2 gebildeten Radius 2.5 ist Schwenkradius und so gewählt, daß der innere Scherarm 2 dem äußeren Scherarm 3, entsprechend der Öffnungsweite des Gelenkes 4 bzw. im Bereich der gelenkbildenden Zusammenführung 4.1 der Scherarme, verschwenkbar ist.

Durch Figur 3 wird die Nahtentfernungsschere in dem angegebenen Schnitt I-I mit einem von der Manipulationsseite 1.1 betrachtet, nach rechts abgebogenen Funktionsende 1.2 dargestellt.

Die Lage der Nahtentfernungsschere 1 entspricht dabei der Anwendung in der Mundhöhle bei Entfernung einer distal hinter dem 5-er-Molar gesetzten Nahtschlinge.

Die Lage der Zähne in der Mundhöhle und die Lage des Manipulationsendes der Nahtentfernungsschere ist gestrichelt dargestellt.

**Patentansprüche**

1. Nahtentfernungsschere, bestehend aus zwei Scherarmen (2,3) mit je einem kurzen Funktionsende, d.h. mit einem kurzen Bereich der gelenkbildenden Zusammenführung (4.1) und einem kurzen Bereich der Schneidenausbildung (2.2, 3.2) und einem langen Manipulationsende (1.1) mit Ösengriff (2.1,3.1), wobei zwischen den im wesentlichen geradlinig verlaufenden Funktions- und Manipulationsenden U-förmige Umlenkbereiche (2.4, 3.4) vorgesehen sind, die winklig bis bogenförmiq ausgeführt sind, so daß das Funktionsende zum Manipulationsende um 180° umgelenkt ausgebildet ist, und wobei das Breitenmaß der beiden Scherarme (2,3) im Bereich des Funktionsendes jeweils um die Hälfte reduziert ausgebildet ist, insbesondere für den Gebrauch in der Mundhöhle, dadurch gekennzeichnet,

daß die Umlenkung um eine Achse erfolgt, die senkrecht zur Bewegungsebene beider Ösengriffe (2.1, 3.1) verläuft, und

daß der Abstand (2.5) des beide Scherarme (2,3) verbindenden Gelenkes (4) zu dem Außenbereich des im Umlenkbereich innenliegenden Scherarmes (2) so gewählt ist, daß im Umlenkbereich der innenliegende Scherarm (2) im außenliegenden Scherarm (3), entsprechend der Öffnungsweite des verbindenden Gelenkes, (4), voll verschwenkbar ist.

2. Nahtentfernungsschere nach Anspruch 1, dadurch gekennzeichnet,

daß die Umlenkung (3.4) des außenliegenden Scherarmes (3) vollständig ist, während die Umlenkung (2.4) des innenliegenden Scherarmes (2) ein geringfügig reduziertes Winkelmaß gegenüber dem Winkelmaß der Umlenkung (3.4) des außenliegenden Scherarmes (3) aufweist, und

daß bei zusammengeführten Manipulationsenden (1.1) und damit bei geschlossenen Funktionsenden, der innere, vom Manipulationsende (1.1) bis zum Umlenkbereich (2.4) führende Teil des innenliegenden Scherarmes (2) zum außenliegenden Scherarm (3) einen spitzen, sich in Richtung auf den Umlenkbereich (2.4) öffnenden Winkel bildet.

3. Nahtentfernungsschere nach Anspruch 1 und 2, dadurch gekennzeichnet,

daß der Drehpunkt des Gelenkes (4) unmittelbar im Auslaufbereich der zum Funktionsende gerichteten Umlenkbereiche (2.4,3.4) der Scherarme (2,3) angeordnet ist.

4. Nahtentfernungsschere nach den Ansprüchen 1 bis 3, dadurch gekennzeichnet, daß das Längenmaß der vom Gelenk (4) zum Manipulationsende (1.1) gerichteten Bereiche der Scherarme (2,3) ein Vielfaches der Länge der das Funktionsende bildenden Scherarmbereiche aufweist.

5. Nahtentfernungsschere nach Anspruch 4, dadurch gekennzeichnet,

daß das Längenverhältnis der Funktionsenden zu den Manipulationsenden (1.1) der Scherarme (2,3) 1:4 bis 1:8 beträgt.

6. Nahtentfernungsschere nach Anspruch 1 bis 4, dadurch gekennzeichnet,

daß bezogen auf die Bewegungsebene der Ösengriffe (2.1,3.1) das Funktionsende (1.2) seitlich abgebogen ist.

**Claims**

1. Scissors for suture removal, consisting of two legs (2, 3) having each a short functional end, i.e. a short section of pivotal junction (4.1), and a short section of blade formation (2.2, 3.2) and a long manipulation end (1.1) with eyed handle (2.1, 3.1), between the essentially straightly extending functional and manipulating ends U-shaped bent sections (2.4, 3.4) of angular to arch form being provided, the functional end thus being bent by 180° with relation to the manipulating end, the width of each of the two legs (2, 3) in the area of the functional end being reduced by half, particularly for use in the oral cavity, characterized in that the bend is around an axis extending vertically to the plane of motion of both eyed handles (2.1, 3.1) , and that the space (2.5) from the joint (4) connecting the two legs (2, 3) to the exterior part of the leg (2) disposed on the inside of the bend is such that in the bend area the inwardly disposed leg (2), according to the spacing of the connecting joint (4), is fully pivotable with in the outwardly disposed leg (3).

2. Scissors for suture removal according to claim 1, characterized in that the bend (3.4) of the outwardly disposed leg (3) is complete while the bend (2.4) of the inwardly disposed leg (2) is of slightly reduced angular dimension as compared to that of the bend (3.4) of the outwardly disposed leg (3), and that, with the manipulating ends (1.1) joined, and thus functional ends closed, the inner part of the inwardly disposed leg (2) extending from the manipulating end (1.1) to the bend area (2.4) forms an acute angle opening towards the bend (2.4).

3. Scissors for suture removal according to claims 1 and 2, characterized in that the fulcrum of the joint (4) is disposed immediately in the final section of the bent areas (2.4, 3.4) of the legs (2, 3) pointing to the functional end.

4. Scissors for suture removal according to claims 1 to 3, characterized in that the longitudinal dimension of the sections of the legs (2, 3) extending from the joint (4) to the manipulating end (1.1) amounts to a multiple of the length of the leg sections forming the functional end.

5. Scissors for suture removal according to claim 4, characterized in that the longitudinal relation of the functional ends as compared to the manipulating ends (1.1) of the legs (2, 3) amounts to 1:4 to 1:8.

6. Scissors for suture removal according to claims 1 to 4, characterized in that the functional end (1.2) is laterally bent off with relation to the plane of motion of the eyed handles (2.1, 3.1)

**Revendications**

1. Ciseaux pour enlèvement de sutures, consistant de deux branches de ciseaux (2, 3) ayant chacune une extrémité de fonction courte, c.à d. une section courte d'assemblage en joint articulé (4.1), et une section courte formée en lame (2.2, 3.2), et une extrémité de manipulation longue (1.1) avec manche en anneau (2. 1, 3. 1), entre les extrémités de fonction et celles de manipulation d'extension essentiellement rectiligne des sections recourbées en U (2.4, 3.4) en forme d'angle à arc étant prévues, de sorte que l'extrémité de fonction en relation à celle de manipulation est recourbée de 180°, la mesure en largeur des deux branches tranchantes (2, 3) dans la section de l'extrémité de fonction étant réduite de moitié, particulièrement pour usage dans la cavité buccale, caractérisés en ce que le recourbement se fait autour d'un axe s'étendant verticalement par rapport au plan de mouvement des deux manches en anneaux (2.1, 3.1), et que la distance (2.5) du joint articulé (4) liant les deux branches tranchantes (2,3) jusqu' à la section extérieure de la branche tranchante (2) disposée à l'intérieur de la section recourbée est choisie telle que dans la section recourbée la branche tranchante (2) disposée à l'intérieur est, selon l'écartement du joint articulé (4), totalement oscillable dans la branche tranchante (3) disposée à l'extérieur.

2. Ciseaux pour enlèvement de sutures selon revendication 1, caractérisés en ce que le recourbement (3.4) de la branche tranchante (3) disposée à l'extérieur est complet, tandis que le recourbement (2.4) de la branche tranchante (2) disposée à l'intérieur est d'une mesure angulaire minimalement réduite comparée à celle du recourbement (3.4) de la branche tranchante (3) disposée à l'extérieur, et qu'en état rapproché des deux extrémités de manipulation (1.1), et donc état fermé des deux extrémités de fonction, la partie intérieure de la branche tranchante (2) disposée à l'intérieur, s'étendant du bout de manipulation (1.1) jusqu'à la section recourbée (2.4), forme avec la branche tranchante (3) disposée à l'extérieur un angle aigu s'ouvrant vers la section recourbée.

3. Ciseaux pour enlèvement de sutures selon les revendications 1 et 2, caractérisés en ce que le centre de rotation du joint articulé (4) est disposé immédiatement dans la partie finale des sections recourbées (2.4, 3.4) des branches tranchantes (2, 3)

4. Ciseaux pour enlèvement de sutures selon les revendications 1 à 3, caractérisés en ce que la mesure de longueur des parties des branches tranchantes (2, 3) s'étendant du joint articulé (4) au bout de manipulation (1.1) est un multiple de la longueur des parties des branches tranchantes formant l'extrémité de fonction.

5. Ciseaux pour enlèvement de sutures selon revendication 4, caractérisés en ce que la relation en longueur entre les extrémités de fonction et celles de manipulation (1.1) des branches tranchantes (2,3) est de 1:4 à 1:8.

6. Ciseaux pour enlèvement de sutures selon les revendications 1 à 4, caractérisés en ce que l'extrémité de fonction (1.2) est courbée latéralement en relation au plan de mouvement des manches en anneaux (2.1, 3.1).

Fig.1

Fig.2

Fig.3
(Schnitt I-I aus Fig.1)